# EUROPÄISCHE PATENTANMELDUNG

(11) **EP 2 740 429 A1**
(43) Veröffentlichungstag der Anmeldung: **11.06.2014**
(21) Anmeldenummer: 12008164.1
(22) Anmeldetag: 06.12.2012
(51) Int. Cl.: A61B 17/88, A61M 5/315, G01F 11/02

(54) **Vorrichtung zur Applikation von Knochenzement**

(71) Anmelder: Heraeus Medical GmbH, 61273 Wehrheim (DE)
(72) Erfinder: Vogt, Sebastian, 99092 Erfurt (DE); Wahnes, Christian, 68167 Mannheim (DE)
(74) Vertreter: Panten, Kirsten

(57) **Zusammenfassung**

Die Erfindung betrifft eine Vorrichtung zur Applikation von Knochenzement. Die Vorrichtung weist einen Hebelarm (2) auf, welcher über ein Gelenk (4) mit einem zweiten Hebelarm (3) verbunden ist. Einer der beiden Hebelarme (3) weist eine nach außen und innen offene Hülse (5) auf, die zur Aufnahme einer Spritze dient. Durch Zusammendrücken der beiden Hebelarme wird der Spritzenkolben kontrolliert in die Spritze gedrückt. Die Vorrichtung reduziert mögliche Anwendungsfehler.

## Beschreibung

Gegenstand der Erfindung ist eine Vorrichtung zur Applikation von Knochenzement in Knochenkavitäten, die insbesondere zum Auffüllen und Stabilisieren von frakturierten Wirbelkörpern bestimmt ist.

Frakturierte Wirbelkörper treten vielfach bei Patienten mit Osteoporose in Form von Kompressionsbrüchen auf. Diese Kompressionsbrüche sind häufig mit erheblichen Schmerzen verbunden. Zur Verminderung der Schmerzen können die frakturierten Wirbelkörper mit Knochenzement aufgefüllt und stabilisiert werden. Als Knochenzemente kommen sowohl Polymethylmethacrylat-Knochenzemente als auch Calciumphosphat-Zemente in Betracht. Es sind als minimal-invasive Operationsverfahren die Kyphoplastie und die Vertebroplastie üblich.

Bei der Kyphoplastie wird ein Ballon in den Wirbelkörper eingebracht und anschlie-βend aufgepumpt. Dabei soll der Wirbelkörper etwas aufgerichtet werden und damit die ventrale Höhenverminderung verringert werden. Anschließend wird der Ballon abgelassen und die im Wirbelkörper entstandene Kavität wird mit Knochenzement ausgefüllt.

Bei der Vertebroplastie wird über eine Hohlnadel Knochenzement in den frakturierten Wirbelkörper nach zuvor erfolgter Entlastung des Wirbelkörpers durch geeignete Lagerung des Patienten gespritzt. Der Knochenzement härtet nach einer entsprechenden Wartezeit aus. Problematisch ist dabei jedoch, dass Knochenzement in die ableitenden Venen fließen kann. Dadurch sind lebensbedrohliche Embolien möglich. Eine weitere ernste Komplikation besteht darin, dass der Knochenzement in den Wirbeikanal gelangt und dort das Rückenmark schädigt. Diese Schädigungen können bis zur Querschnittslähmung führen. Zur Vermeidung oder Verminderung dieser Probleme erfolgt das Einspritzen des Knochenzements unter Röntgenkontrolle. Aus diesem Grund werden für die Vertebroplastie bevorzugt Polymethylmethacrylat-Zemente mit einem sehr hohen Anteil an Röntgenopaker, wie zum Beispiel Zirkoniumdioxid, verwendet. Ein Beispiel dafür ist der speziell für die Vertebroplastie vorgesehene Zement Osteopal® V der Firma Heraeus Medical GmbH (Wehrheim), der 40 % Zirkoniumdioxid im Zementpulver enthält. Durch den hohen Anteil an Röntgenopaker ist es möglich, das Einspritzen des Zementes röntgenologisch leicht zu verfolgen. Das bedeutet, die gesamte Einspritzphase des Knochenzementes wird röntgenologisch unter Sicht (Fluoroskopie) kontrolliert, um die Zementeinspritzung sofort zu beenden, falls es erste Anzeichen des unerwünschten Austritts des Knochenzements aus dem Wirbelkörper gibt.

Zur weiteren Verminderung des unerwünschten Zementaustritts aus dem frakturierten Wirbelkörper ist es sinnvoll, hochviskose Zemente einzusetzen, die eine geringe Tendenz zum Fließen in der Verarbeitungsphase haben. Problematisch ist jedoch dabei, dass diese Zemente zähflüssig und nur mit großem Kraftaufwand spritzbar sind. Aus diesem Grund wurde eine Reihe von Vorrichtungen vorgeschlagen, die eine Applikation des Knochenzementes für den medizinischen Anwender erleichtern.

Im US200210099374 A1 wird eine Auspressvorrichtung beschrieben, die aus einer Kartusche mit einem Austragsrohr und zwei Kolben besteht, wobei der zweite Kolben im ersten Kolben angeordnet ist und dazu dient, den Zement nach Auspressen aus der Kartusche im Austragsrohr weiter voran zu treiben.

Alternativ zu diesen relativ einfachen Auspressvorrichtungen wurde vorgeschlagen, Kolben mit Gewinden durch Drehung in Kartuschen mit Innengewinden oder Außengewinden zum Auspressen einzusetzen (US 6,348,055 B1, EP 1 074 231 A1, US 6, 383,190 B1, US2003/0040718 A1).

Weiterhin wurden Kombinationen von Schraubenvortrieben und hydraulischen Systemen offenbart (EP 1 614 403 A1).

Eine Weiterentwicklung stellen reine hydraulische Auspressvorrichtungen dar (WO 2010/006436 A1).

In US 2002/0156483 A1 wird eine Auspressvorrichtung beschrieben, die aus zwei Hebeln besteht, die über Gelenke miteinander verbunden sind. Ein Hebel ist als Zementkartusche ausgebildet und enthält einen Kolben, mit dem Zement in einer senkrecht zur Kartusche angeordnete Kammer gespritzt werden kann. Durch Bewegung der Hebel zueinander wird mit einem in der Kammer angeordneten Stößel der Zement ausgedrückt. Der Aufbau der Auspressvorrichtung ist relativ komplex. Problematisch ist bei dieser Vorrichtung, dass der Zement in den als Kartusche ausgebildeten Hebel eingefüllt werden muss und dass jeweils der Zement portionsweise durch den Kolben der Kartusche in die Kammer übertragen werden muss.

Die Aufgabe der Erfindung ist eine Auspressvorrichtung zu entwickeln, die einfacher zu befüllen ist, wobei hochviskose Knochenzemente mit geringem Kraftaufwand kontrolliert appliziert werden sollen. Die Betätigung der Vorrichtung soll so erfolgen, dass der medizinische Anwender sich während des Applizierens nicht mit seinen Händen im Röntgenstrahl befindet und damit die Strahlenbelastung vermindert wird.

Diese Aufgabe wird durch eine Vorrichtung mit den Merkmalen des Anspruchs 1 gelöst. Weiterbildungen der Erfindung sind durch die Unteransprüche definiert.

Erfindungsgemäß weist die Vorrichtung zum Applizieren von Knochenzement einen Hebelarm auf, welcher über ein Gelenk mit einem zweiten Hebelarm verbunden ist, wobei einer der Hebelarme eine nach außen und innen offene Hülse zur Aufnahme einer medizinischen Spritze aufweist. Hier bedeutet der Ausdruck "innen" der Raum zwischen den beiden Hebelarmen, während der Ausdruck "außen" den Raum außerhalb der beiden Hebelarme bezeichnet.

Bevorzugt handelt es sich bei dem Gelenk um ein Scharniergelenk. Besonders bevorzugt wird der Bolzen des Scharniergelenks aus zwei miteinander verrastbaren Teilbolzen gebildet. Bei der Ausführung der Vorrichtung in Polyamid oder glasfaserverstärkten Polyamid können vorteilhaft in einander steckbare, verrastbare Bolzen zur Ausbildung des Scharniergelenks genutzt werden. Die Montage ist durch einfaches Ineinanderstecken der Bolzen möglich.

Neben einem Scharniergelenk sind weitere Beispiele für geeignete Gelenke ein Foliengelenk, ein Kugelgelenk, ein Ellipsengelenk und ein Sattelgelenk.

Die Hülse hat einen Innendurchmesser, der größer oder gleich dem Außendurchmesser einer medizinischen Spritze ist, wobei die Hülse bevorzugt eine Länge von einem Drittel bis der ganzen halben Länge einer medizinischen Spritze hat. Auf diese Weise sitzt die Spritze sicher in der Hülse. Vorteilhaft hat die Hülse mindestens die Halbe Länge des Spritzenzylinders.

Der Begriff Spritze umfasst alle üblichen aus Kunststoff und Glas oder sonstigen Materialien gefertigten Spritzen. Bevorzugt handelt es sich hier um Spritzen aus Kunststoff mit einem Volumen von maximal 60 ml, bevorzugt 30 bis 50 ml.

Es ist von Vorteil, wenn der der Hülse gegenüberliegende Hebelarm eine Einrichtung zur Fixierung des Spritzenkolbens aufweist Hierbei kann es sich einfach um eine Mulde handeln, in die der Spritzenkolben hineinragt. Bevorzugt ist die Einrichtung zur Fixierung des Spritzenkolbens in einem Stempel ausgebildet. Besonders bevorzugt hat der Stempel ein Außengewinde und ist in einer Bohrung mit Innengewinde im Hebelarm drehbar angeordnet. Das bedeutet, dass, nachdem der Knochenzement teilweise aus der Spritze herausgepresst worden ist, der Stempel gedreht werden kann, so dass sich die Vertiefung in Bezug auf den Hebelarm verschiebt. Dadurch wird die ursprüngliche Entfernung der Hebelarme wiederhergestellt, so dass sie wieder gut vom Benutzer gefasst werden können und ideal Druck ausgeübt werden kann.

Die in dem Stempel ausgebildete Vertiefung oder Mulde kann auch glockenförmig ausgebildet sein. Die Vertiefung hat einen Innendurchmesser von mindestens dem Außendurchmesser des Endes eines Spritzenkolbens. Dadurch wird gewährleistet, dass der Kolben sicher in der Aufnahmeeinheit gelagert werden kann und beim Auspressen der Spritze durch Bewegung der Hebelarme gegeneinander der Kolben ausreichend fixiert ist und der Bewegung der Hebelarme nicht ausweichen kann.

Von besonderem Vorteil ist es, wenn zwischen beiden Hebelarmen eine gewisse, dem Druck der Bedienperson entgegengesetzte Rückstellkraft vorhanden ist. Dies erfolgt vorteilhaft durch ein elastisches Bauelement, wie eine Spiralfeder oder Blattfeder. Mit dem elastischen Element wird erreicht, dass einerseits eine genaue Kontrolle des auf die Spritze ausgeübten Drucks durch die eingesetzte Handkraft möglich ist und dass andererseits der Pressvorgang problemlos angehalten werden kann.

Das Begrenzungselement ist bevorzugt als Zahnstange oder als Spindel ausgebildet. Durch das Begrenzungselement kann die Strecke der Bewegung der Hebelarme und damit das Volumen des auszupressenden Knochenzementes vorgegeben werden. Typische Zementvolumina bei der Vertebroplastie sind 2 ml bis 3 ml Knochenzement pro Wirbelkörper. Es ist auch möglich, durch Wahl eines geeigneten Zahnabstandes der Zahnstange Rastelemente nach jeweils 0,1 ml vorzusehen. Damit kann vermieden werden, dass zu große Zementmengen ausgepresst werden. Es ist auch möglich, an dem Begrenzungselement visuell oder auch haptisch wahrnehmbare Markierungen anzubringen, die es dem medizinischen Anwender erlauben, das applizierte Zementvolumen zu kontrollieren. Neben Zahnstangen können auch Spindeln als Begrenzungselemente mit Vorteil eingesetzt werden. Dabei ist es jedoch erforderlich, in einem der Hebelarme mindestens eine Bohrung mit Gewinde für die Aufnahme der Spindel anzuordnen.

Zur Applikation des Knochenzementes wird der Knochenzement in eine Spritze mit Kolben gefüllt. Nachdem die beiden Hebelarme der Vorrichtung auseinandergezogen worden sind, wird die Spritze in die in einem Hebelarm der Vorrichtung ausgebildete Hülse eingesetzt. Anschließend werden beide Hebelarme zusammengedrückt, bis das Ende des Spritzenkolbens sicher in der Einrichtung zur Fixierung des Spritzenkolbens angeordnet ist. Der Anwender drückt beide Hebelarme gegeneinander, bis Knochenzement aus der Spritzendüse austritt. Die erforderliche Menge an Knochenzement wird dabei eingestellt, indem die Hebelarme über die entsprechende Anzahl Rastelemente gedrückt werden.

Die Vorrichtung zur Applikation von Knochenzement sollte möglichst nicht röntgenopak sein. Die Vorrichtung ist bevorzugt aus Kunststoff ausgebildet. Besonders bevorzugt sind aliphatische und/oder aromatische Polyamide, glasfaserverstärkte aliphatische und/oder aromatische Polyamide, Polyimide, glasfaserverstärkte Polyimide, Polyetherketon und Polysulfon. Besonders gut geeignete Materialien sind zum Beispiel GRILAMID TR95 und GRIVORY G der Firma EMS-GRIVORY. Daneben kann die Vorrichtung aus Epoxid-Harzen, Phenolharzen oder Harnstoffharzen gefertigt werden. Es ist auch möglich, aus Aluminiumlegierungen, Magnesiumlegierungen oder aus Kunststoff-Metall-Kompositen die Vorrichtung herzustellen.

Erfindungsgemäß ist auch ein Kit, der aus der Vorrichtung zur Applikation von Knochenzement, einer Spritze, mindestens einer Hohlnadel und mindestens einem verformbaren hohlen Verbindungselement zusammengesetzt ist, das den Spritzenkopf und die Hohlnadel verbindet. Bevorzugt weisen die Spritze an der Spritzendüse und die Spritzenkanüle Gewinde auf, die zu Gewinden des verformbaren hohlen Verbindungselementes passen. Über die Gewinde kann das Verbindungselement zwischen Spritzendüse und Spritzenkanüle eingesetzt werden. Auf diese Weise wird beim Bewegen der Applikationsvorrichtung keine Kraft auf die Kanüle und damit auf den zu behandelnden Knochen ausgeübt.

Somit wird eine Vorrichtung bereitgestellt, die mit geringem Aufwand kostengünstig zu fertigen ist. Die Vorrichtung kann aus einer geringen Anzahl von Teilen aufgebaut sein und ist somit so unkompliziert und einfach aufgebaut, dass eine leichte und sichere Bedienung gewährleistet ist und Anwendungsfehler minimiert werden.

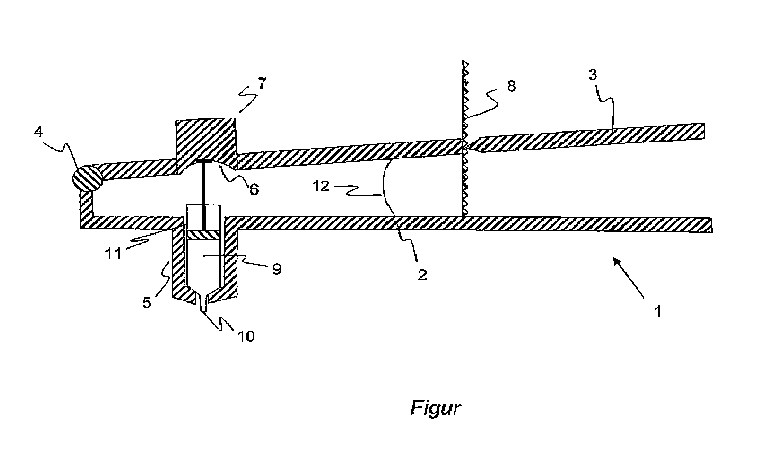

Im Folgenden wird eine bevorzugte Ausführungsform der vorliegenden Erfindung anhand der beiliegenden Zeichnung genauer beschrieben.

In der Figur ist eine Vorrichtung 1 zum Applizieren von Knochenzement dargestellt. Die Vorrichtung enthält zwei Hebelarme 2,3, die über ein Gelenk 4 miteinander verbunden sind. In einem der beiden Hebelarme 3 ist eine Hülse 5 ausgebildet. Die Hülse 5 weist nach innen hin eine Öffnung 11 auf, die eine Spritze 9 aufnimmt. Am äußeren Ende weist die Hülse 5 eine zweite Öffnung auf, die größer als die Spritzendüse 10, jedoch kleiner als der Spritzenzylinder ist. Auf diese Weise sitzt der Spritzenzylinder sicher in der Hülse 5, während die Spritzendüse 10 durch die zweite, die kleinere Öffnung ragt. Durch diese Düse 10 wird der Zement, der sich in der Spritze 9 befindet, appliziert.

Der Hülse 5 gegenüberliegend ist in den zweiten Hebelarm 3 ein Gewinde eingelassen, in das ein Stempel 7 geschraubt ist. Dieser Stempel 7 weist eine muldenförmige Vertiefung 6 auf, die den Spritzenkolben am äußeren Ende aufnimmt, Indem der Anwender die beiden Hebelarme 2,3 zusammendrückt, wird der Spritzenkolben in die Spritze gedrückt und Knochenzement aus der Düse 10 herausgedrückt.

Zwischen den beiden Hebelarmen 2,3 ist außerdem eine Zahnstange 8 angeordnet, welche Unterteilungen hat, die jeweils zum Beispiel einer Ausgabe von Knochenzement von etwa 0,1 ml entsprechen. Durch eine Blattfeder 12 werden die Hebelarme auseinandergedrückt.

### Bezugszeichenliste

- 1: Vorrichtung zur Applikation von Knochenzement
- 2: Hebelarm
- 3: Hebelarm
- 4: Gelenk
- 5: Hülse
- 6: Mulde
- 7: Stempel
- 8: Zahnstange
- 9: Spritze
- 10: Spritzendüse
- 11: Öffnung
- 12: elastisches Element, Blattfeder

## Patentansprüche

1. Vorrichtung zum Applizieren von Knochenzement **gekennzeichnet durch** einen Hebelarm (2), welcher über ein Gelenk (4) mit einem zweiten Hebelarm (3) verbunden ist, wobei einer der beiden Hebelarme (3) eine nach außen und innen offene Hülse (5) zur Aufnahme einer Spritze aufweist.

2. Vorrichtung nach Anspruch 1, **dadurch gekennzeichnet, dass** der zweite Hebelarm (3) eine Einrichtung (6) zur Fixierung des Spritzenkolbens aufweist, welche der Hülse (5) gegenüberliegend angeordnet ist.

3. Vorrichtung nach Anspruch 2, **dadurch gekennzeichnet, dass** die Einrichtung (6) zur Fixierung des Spritzenkolbens eine Mulde am zweiten Hebelarm (3) ist.

4. Vorrichtung nach einem der Ansprüche 2 oder 3,
**gekennzeichnet durch**
einen Stempel (7) aufweist, der bewegbar in dem zweiten Hebelarm (3) angeordnet ist, und der die Einrichtung (6) zur Fixierung des Spitzenkolbens aufweist.

5. Vorrichtung nach Anspruch 4,
**dadurch gekennzeichnet, dass**
der Stempel ein Außengewinde aufweist und in eine Bohrung mit Innengewinde des zweiten Hebelarms (3) drehbar angeordnet ist.

6. Vorrichtung nach einem vorhergehenden Ansprüche, **dadurch gekennzeichnet, dass**
das Gelenk (4) einen Bolzen aufweist, der aus zwei miteinander verrastbaren Teilbolzen gebildet wird.

7. Vorrichtung nach einem vorhergehenden Ansprüche, **dadurch gekennzeichnet, dass**
die Hülse (5) einen solchen Innendurchmesser hat, der geringfügig größer oder gleich dem Außendurchmesser einer medizinischen Spritze (10) ist und eine Länge von einem Drittel der Länge bis zur ganzen Länge der medizinischen Spritze (10) hat.

8. Vorrichtung nach einem der vorhergehenden Ansprüche, **dadurch gekennzeichnet, dass**
die Hülse (5) eine nach außen weisende Öffnung hat, deren Durchmesser geringer als der Durchmesser des zylindrischen Hohlkörpers der Spritze (9) jedoch größer als die Spritzendüse (10) ist.

9. Vorrichtung nach einem vorhergehenden Ansprüche, **dadurch gekennzeichnet, dass**
zwischen den beiden Hebelarmen (2,3) ein elastisches Element (12) angeordnet ist.

10. Vorrichtung nach Anspruch 9, **dadurch gekennzeichnet, dass** das elastische Element (12) als Spiralfeder oder als Blattfeder ausgebildet ist.

11. Vorrichtung nach einem der vorhergehenden Ansprüche, **gekennzeichnet durch**
mindestens ein Begrenzungselement (8), das die Strecke der Bewegung der beiden Hebelarme (2,3) zueinander definiert.

12. Vorrichtung zum Applizieren von Knochenzement nach einem der Ansprüche 9, **dadurch gekennzeichnet, dass**
das Begrenzungselement (8) als Zahnstange oder als Spindel ausgebildet ist.

13. Vorrichtung nach einem der vorhergehenden Ansprüche, **dadurch gekennzeichnet, dass**
die Vorrichtung aus (1) Kunststoff besteht.

14. Vorrichtung nach Anspruch 13, **dadurch gekennzeichnet, dass** es sich bei dem Kunststoff um einen oder mehrere handelt, die aus der Gruppe ausgewählt sind, die aus aliphatischen und aromatischen Polyamiden, glasfaserverstärkten aliphatischen und aromatischen Polyamiden, Polyimiden, glasfaserverstärkten Polyimiden, Polyetherketon und Polysulfon besteht.
